# EUROPEAN PATENT SPECIFICATION

(11) **EP 1 668 371 B1**
(45) Date of publication and mention of the grant of the patent: **09.05.2012**
(21) Application number: 04773337.3
(22) Date of filing: 14.09.2004
(51) Int. Cl.: G01N 33/68

(54) **METHOD OF DIAGNOSING OVARIAN ENDOMETRIOSIS USING TFPI-2 PROTEIN**
VERFAHREN ZUR DIAGNOSE VON ENDOMETRIOSE MITTELS TFPI-2 PROTEIN
METHODE DE DIAGNOSTIC D'ENDOMETRIOSE OVARIENNE A L'AIDE D'UNE PROTEINE TFPI-2

(30) Priority: 24.09.2003 US 505572 P
(43) Date of publication of application: 14.06.2006
(73) Proprietor: Oncotherapy Science, Inc., Kawasaki-shi Kanagawa 213-0012 (JP)
(72) Inventor: NAKAMURA, Yusuke, Yokohama-shi, Kanagawa 2250011 (JP); KATAGIRI, Toyomasa, Shinagawa-ku, Tokyo 1410022 (JP)
(74) Representative: Vossius & Partner
(86) International application number: PCT/JP2004/013718
(87) International publication number: WO 2005/029089

(56) References cited:
- WO-A-2004/024952
- DATABASE BIOSIS [Online] BIOSCIENCES INFORMATION SERVICE, PHILADELPHIA, PA, US; 1982, INABA N ET AL: "IMMUNO HISTOCHEMICAL DETECTION OF PREGNANCY SPECIFIC PROTEIN SP-1 AND PLACENTA SPECIFIC TISSUE PROTEINS PP-5 PP-10 PP-11 AND PP-12 IN OVARIAN ADENO CARCINOMAS" XP002321429 Database accession no. PREV198376027239 & ONCODEVELOPMENTAL BIOLOGY AND MEDICINE, vol. 3, no. 5-6, 1982, pages 379-390, ISSN: 0167-1618
- BUTZOW R ET AL: "IMMUNOFLUOROMETRIC DEMONSTRATION AND QUANTIFICATION OF PLACENTAL PROTEIN 5 IN THE ABSENCE OF PREGNANCY" CLINICAL CHEMISTRY, vol. 34, no. 8, 1988, pages 1591-1593, XP002321428 ISSN: 0009-9147

### SEQUENCE LISTING

<110> ONCOTHERAPY SCIENCE. INC.
   THE UNIVERSITY OF TOKYO
<120> Method of diagnosing ovarian endometriosis using TFPI-2 protein
<130> ONC-A0304P
<150> US 60/505,572
   <151> 2003-09-24
<160> 5
<170> Patentin version 3.1
<210> 1
   <211> 22
   <212> DNA
   <213> Artificial
<220>
   <223> artificially sinthesized primer sequence for RT-PCR
<400> 1
   tgacagcatg aggaaacaaa tc 22
<210> 2
   <211> 20
   <212> DNA
   <213> Artificial
<220>
   <223> Artificially synthesized primer sequence for RT-PCR
<400> 2
   acgaccccaa gaaatgagtg 20
<210> 3
   <211> 20
   <212> DNA
   <213> Artificial
<220>
   <223> Artificially synthesized primer sequence for RT-PCR
<400> 3
   cgaccacttt gtcaagctca 20
<210> 4
   <211> 23
   <212> DNA
   <213> Artificial
<220>
   <223> Artificially synthesized primer sequence for RT-PCR
<400> 4
   ggttgagcac agggtacttt att 23
<210> 5
   <211> 20
   <212> DNA
   <213> Artificial
<220>
   <223> Artificially synthesized primer sequence for northern hybridization
   on
<400> 5
   ggaaaattcg gaagaagcaa 20

## Description

### FIELD OF THE INVENTION

Tissue factor pathway inhibitor-2 (IFPI-2) is shown herein to be potential diagnostic and therapeutic target for endometriosis. Thus, the present invention relates to methods of diagnosing ovarian endometriosis using TFPI-2 protein as an index, and the use of kits for the diagnosis of ovarian endometriosis.

### BACKGROUND OF THE INVENTION

Endometriosis is a common gynecologic disorder, affecting at least 10% of women of reproductive age (Rice (2002) Ann. N.Y. Acad. Sci. 955: 343-352). Endometriosis is defined as the presence of endometrial glands and stroma outside the uterus. Its most common symptoms are progressive dysmenorrhea, dyspareunia, chronic pelvic pain and infertility Endometriosis with ovarian cysts can be diagnosed through techniques such as ultrasonography or magnetic resonance image (MRI); however, endometriosis without ovarian cysts is difficult to diagnose without surgical invasion (Rice (2002) Ann. N.Y Acad Sci. 955: 343-52) since the symptoms mentioned above are not specific to endometriosis. For example, though the concentration of CA125, one of the tumor markers for ovarian carcinoma, is sometimes elevated in the sera of women with endometriosis, due to its low specificity (Evers et al. (1995) "Progress Management of Endometriosis" ed. Cautinho, Parthenon Publishing Groups, Carnforth, 175-84), it is not a very useful marker for the disorder. Furthermore, for a patient whose chief complaint is dysmenorrhea who shows no physical abnormality, it is difficult to distinguish endometriosis from idiopathic dysmenorrhea.

Treatment for endometriosis commonly involves surgical resection and/or medication with hormonal agents, such as gonadotropin-releasing hormone (GnRH) agonists and androgens. However, since long-term medication is undesirable due to side effects such as menopausal disorders (hot flushes and stiff shoulders), genital bleeding, and bone deminemlization, clinical control of endometriosis is often very difficult. Current methods for the treatment of the disease are also problematic in that they are associated with a high recurrence rate. Specifically, the recurrence rate five years after operation and medical treatment has been reported to be about 20% (Redwine (1991) FertiL Steril. 56: 628-34) and as much as 53% (Waller and Shaw (1993) Fertil. Steril. 59: 511-5), respectively.

Various studies of endometriosis have revealed little in terms of the underlying genetic and pathophysiologic mechanisms due to following reasons: (i) epithelial cells of endometrial cysts often wither and peel off such that it is very difficult to obtain sufficient sample material; and (ii) the effect of contamination of the stromal cells under the usual sampling methods is substantial. To overcome these problems, the present inventors scraped and microdissected epithelial cells from the cysts and obtained them with high purity in a forgoing study (Jimbo et al. (1997) Am. J. Pathol. 150: 1173-8).

cDNA microarray technologies have facilitated the construction of comprehensive profiles of gene expression in normal and malignant cells, and the comparison of gene expression in malignant and corresponding normal cells (Okabe et al. (2001) Cancer Res. 61:2129-37; Kitahara et al. (2001) Cancer Res. 61: 3544-9; Lin et al. (2002) Oncogene 21:4120-8; Hasegawa et al. (2002) Cancer Res. 62:7012-7). This approach enables the disclosure of the complex nature of cancer cells, and assists in understanding the mechanism of carcinogenesis. Identification of genes that are deregulated in tumors can lead to more precise and accurate diagnosis of individual cancers as well as the development of novel therapeutic targets (Bienz and Clevers (2000) Cell 103:311-20). Medical applications of microarray technologies include: (i) discovery of genes that contribute to tumorigenesis; (ii) discovery of useful diagnostic biomarker(s) and novel molecular target(s) for anti-cancer agents; and (iii) identification of genes involved in conferring chemosensitivity. Recently, molecules associated with the development of certain types of cancers identified by microarray technologies have been clinically proven to be good targets for developing effective novel drugs for cancers. In an effort to discover mechanisms underlying tumors from a genome-wide point of view and target molecules for diagnosis and development of novel therapeutic agents, the present inventors have used a microarray of cDNAs representing 23,040 human genes to analyze expression profiles of tumors from various tissues (Okabe et al. (2001) Cancer Res. 61: 2129-37; Hasegawa S. et al. (2002) Cancer Res. 62: 7012-7; Kaneta et al. (2002) Jpn. J. Cancer Res. 93: 849-56; Kitahara et al. (2002) Neoplasia 4: 295-303; Lin et al. (2002) Oncogene 21: 4120-8; Nagayama S. et al. (2002) Cancer Res. 62: 5859-66; Okutsu et al. (2002) Mol. Cancer Ther. 1: 1035-42; Kikuchi et al. (2003) Oncogene 22:2192-205). For example, by analyzing expression profiles of hepatocellular carcinomas (HCC), the present inventors discovered *VANGLI,* a gene frequently up-regulated in tumor cells, and demonstrated that suppression of its expression with antisense-oligonucleotides significantly decreased the growth of HCC cells and induced apoptotic cell death (Yagyn et al. (2002) Int. J. Oncol. 220: 1173-8).

Endometriosis is similar to malignant tumors in some respects. For example, endometriotic cells can be both locally and distantly metastatic. In other words, endometriotic cells can adhere, invade and damage other tissues in a manner similar to cancerous cells. Thus, to elucidate the nature of endometriosis, the present inventors carried out cDNA microarray analysis (Arimoto et aL (2003) Int. J. Oncol. 22: 551-60). By comparing expression patterns between endometriotic tissues and corresponding eutopic endometria using genome-wide cDNA microarray analyses, the present inventors identified several genes that were commonly up-regulated in endometrial cysts.

Tissue factor pathway inhibitor-2-(TFPI-2), also known as placental protein 5 (PP5), is a serine proteinase inhibitor containing three (3) tandemly arranged Kunitz-type proteinase inhibitor domains that is homologous to tissue factor pathway inhibitor (Sprecher et al. (1994) Proc. Natl. Acad. Sci. USA 91: 3353-7; Rao et al. (1996) Arch. Biochem. Biophys. 335: 82-92; Miyagi et al. (1994) J. Biochem. 116:939-42). The protein is constitutively secreted from cells of several endothelial cell types (lino et al. (1998) Arterioscler. Thromb. Vasc. BioL 18: 40-6) in three alternatively glycosylated isoforms of 27, 31 and 33 kDa (Rao et al. (1996) Arch. Biocbem. Biophys. 335: 82-92). TFPI-2 is a strong inhibitor of plasmin as well as of trypsin, chymotiypsin, plasma kallikrein, cathepsin G, factor VIIa and factor XIa, but not urokinase-type plasminogen activator (uPA), tissue plasminogen activator or thrombin (Sprecher et al. (1994) Proc. Natl. Acad. Sci. USA 91: 3353-7; Rao et al. (1995) Arch. Biochem. Biophys. 319: 55-62; Rao et al. (1995) Arch. Biochem. Biophys. 317:311-4; Rao et al. (1995) J. Invest. Dermatol. 104: 379-83; Peterson et al. (1996) Biochemistry 35: 266-72). Quantification of this gene using cell-conditioned-medium, extracellular matrix (ECM) and cytoplasmic fractions revealed that most of the proteins encoded by the gene are present in the ECM (Rao et al. (1996) Arch. Biochem. Biophys. 335: 82-92). Furthermore, Neaud *et al*. reported that TFPI-2 potentiates hepatocyte growth factor-induced invasion of hepatocellular carcinoma cells on its own (Neaud et al. (2000) J. Biol. Chem. 275: 35565-9), whereas the expression of TFPI-2 was shown to inversely correlate during the progression of human glioma (Rao et al. (2001) Clin. Cancer Res. 7: 570-6). Various gynecological studies about TFPI-2 have been performed in terms of the progression of pregnancy. However, to date, there is no report that TFPI-2 is associated with the development of endometriosis.

Invasion is a characteristic feature of endometriosis. According to the transplantation theory, endometriosis develops from endometrial fragments that are retrogradely menstruated into the peritoneal cavity. In order to develop into endometriotic lesions, these fragments must attach to the subperitoneal space and invade by interactions with ECM proteins. The proteolytic pathway of invasion depends on the balance of a lot of components including serine proteases, such as uPA and plasmin, matrix metallopmteinases and protease inhibitors (Shapiro (1998) Curr. Opin. Cell Biol. 10: 602-8; Toi et al. (1998) Breast Cancer Res. Treat. 52: 113-24; Andreasen et al. (1997) Int. J. Cancer 72: 1-22). Although the role of ECM-associated TFPI-2 is unclear, since TFPI-2 is primarily found in ECM, TFPI-2 may be important in the regulation of matrix turnover by serine proteases. Moreover, Neaud *et al.* reported that TFPI-2 induces an invasive activity in three different human hepatocellular carcinoma cell lines and stable transfectants (Neaud et al. (2000) J. BioL Chem. 275: 35565-9). On the contrary, it has been demonstrated that TFPI-2 strongly inhibits the *in vitro* invasion of highly invasive HT1080 cell line (Rao et al. (1998) Int. J. Cancer 76: 749-56) and that its expression inversely correlates during the progression of human glioma (Rao et al. (2001) Clin. Cancer Res. 7: 570-6). As reported by Neaud *et al*., TFPI-2 may have both an indirect anti-invasive effect and a direct pro-invasive effect. Other protease inhibitors, for example, plasminogen activator inhibitor-1, also share a dual effect (Deng et al. (1996) J. Cell Biol. 134:1563-71). The interesting phenomenon that an endometriotic cell can be both metastatic and invasive while the disease remains a benign disorder may be explained by the fact that TFPI-2 may have such a dual effect and controls the invasion of endometriotic cells.

### SUMMARY OF THE INVENTION

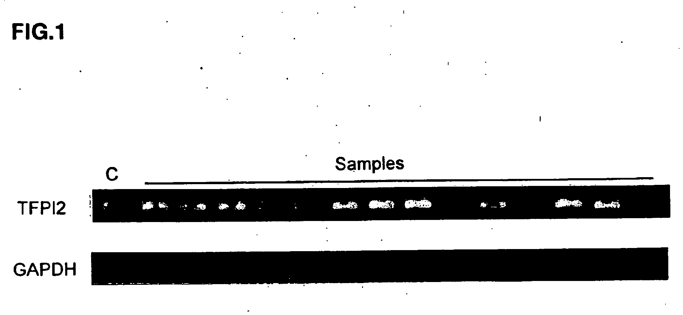

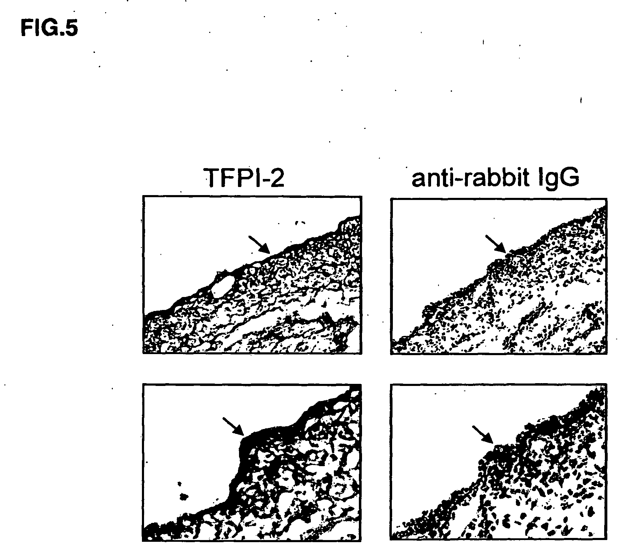

The present invention is based on the discovery that the expression of certain genes correlates with ovarian endometriosis. More particularly, through semi-quantitative RT-PCR and immunohistochemistry (Fig. 1 and 5), the present inventors have discovered that tissue factor pathway inhibitor-2 (TFPI-2) gene is overexpressed in endometrial cysts.

Accordingly, the present invention provides a method of diagnosing endometriosis in a subject by determining the expression level of TFPI-2 protein, in a subject-derived biological sample, and then comparing the detected protein expression level to a control level according to claim 1.

In the context of the present invention, the phrase "control level" refers to a protein expression level detected in a control sample and includes both a normal control level and an ovarian endometriosis control level. A control level may be a single expression pattern derived from a single reference population or may be derived from a plurality of expression patterns. For example, the control level can be a database of previously tested expression patterns. A normal healthy individual is one with no clinical symptoms of ovarian endometriosis and a normal control level includes a protein expression level of TFPI-2 detected in such a normal healthy individual or in a population of individuals known not to suffer from ovarian endometriosis. For the comparison purposes, the normal control level is typically based on, for example, the expression level of TFPI-2 in the normal healthy subject. Based on this normal control level, a permissible range is set, for example, to be the standard value ± 2 S.D. The method of setting up a normal control level and permissible range based on measured values of a diagnostic marker protein is well-known in the art. For example, the normal control level of TFPI-2 of the present invention is less than 20 ng/mL serum, preferably less than 10 ng/mL serum, more preferably less than 5 ng/mL serum. On the other hand, an ovarian endometriosis control level comprises a protein expression level of TFPI-2 detected in a patient diagnosed with ovarian endometriosis.

An alteration, e.g., an increase in the protein expression level of TFPI-2 as compared to a control level, indicates that the subject suffers from ovarian endometriosis. Specifically, when the expression level in a subject sample is increased as compared to a normal control level, the subject is indicated as suffering from ovarian endometriosis. Furthermore, an increase or similarity in the protein expression level of TFPI-2 as compared to an ovarian endometriosis control level indicates that the subject suffers from ovarian endometriosis.

According to the present invention, an expression level of TFPI-2 may be determined as being altered when its expression level increases 10%, 25%, 50% or more as compared to the control level. Alternatively, the expression level of TFPI-2 may be determined as being altered when its expression level increases 1, 2, 5 or more folds as compared to the control level.

The subject-derived biological sample used in the present invention is a blood or blood-derived sample . Examples of blood-derived samples include serum and plasma.

The present invention also provides the use of a kit for the in vitro diagnosis of ovarian endometriosis according to claims 2-7.

Unless otherwise defined, all technical and scientific terms used herein have the same meaning as commonly understood by one of ordinary skill in the art to which the present invention belongs. Although methods and materials similar or equivalent to those described herein can be used in the practice or testing of the present invention, suitable methods and materials are described below. In case of conflict, the present specification, including definitions, will control. In addition, the materials, methods and examples are illustrative only and not intended to be limiting.

Other features and advantages of the invention will be apparent from the following detailed description and from the claims.

### BRIEF DESCRIPTION OF THE DRAWINGS

Figure 1 depicts the expression of TFPI-2 in the secretory phase. The expression of TFPI-2 and GAPDH was examined by semi-quantitative RT-PCR using cDNA prepared from amplified RNA. "C"-indicates control. The results depicted in Fig.1 demonstrate that the expression of TFPI-2 is up-regulated in 9 of 14 cases in the secretory phase.

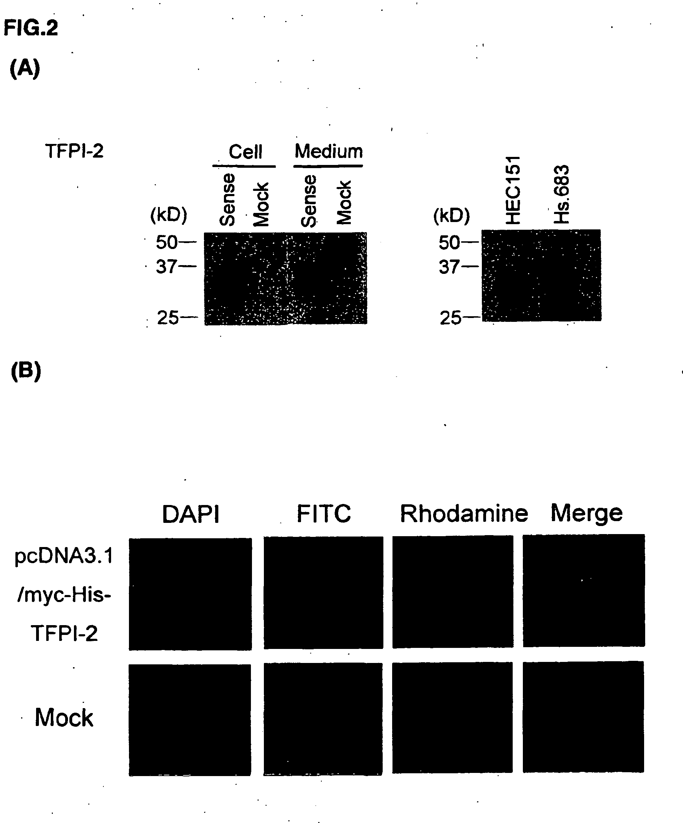

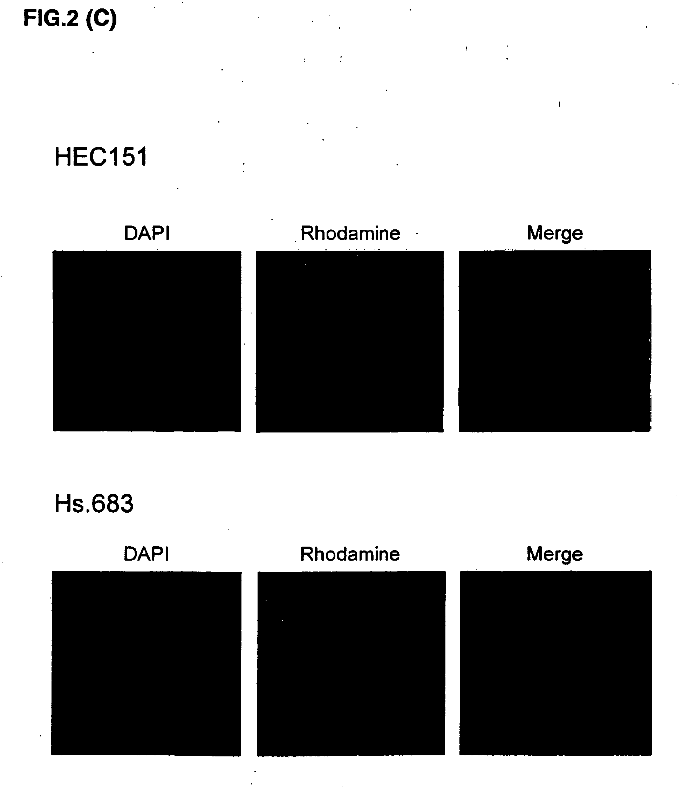

Figure 2 depicts the sub-cellular localization of the TFPI-2 protein. Fig. 2(A) depicts the results of Western blot analysis of myc-tagged TFPI-2 protein using extracts of COS-7 cells transfected with pcDNA3.1-myc/His-sense plasmid or mock. "Cell" refers to cell lysate; and "Medium" refers to conditioned medium. In addition, endogenous expression of TFPI-2 was detected in HEC-151 and Hs.683 cells. Fig. 2(B) depicts the results of immunofluorescent staining of TFPI-2- and Mock- stable transfectants. Fig. 2(C) depicts the results of immunofluorescent staining of HEC-151 and Hs. 683 cells with anti-TFPI-2 antibody. Both exogenous and endogenous expression patterns of TFPI-2 were identical.

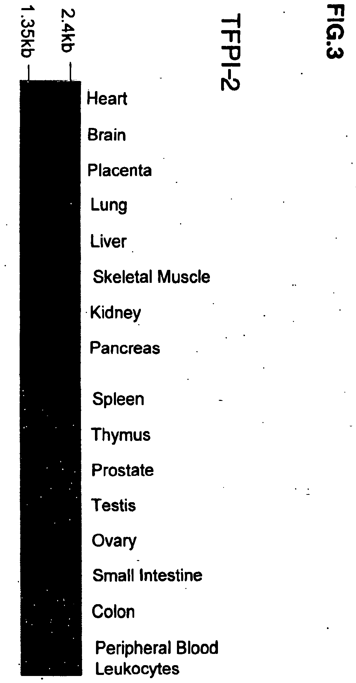

Figure 3 depicts the results of Northern bloc analysis of TFPI-2 mRNAs. Multiple-tissue Northern blot membranes (Clontech) were hybridized with cDNA fragments of TFPI-2 as described under the item "Materials and methods". RNA size markers are indicated in kilobase pairs (kb).

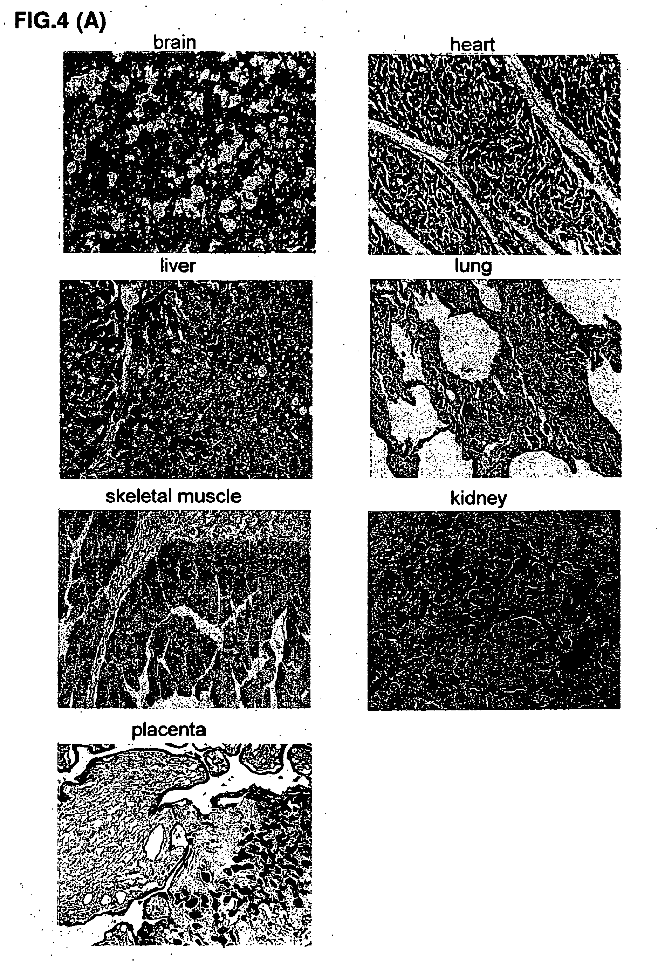

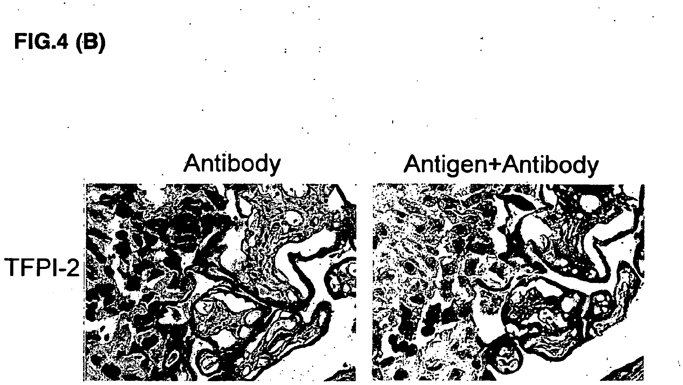

Figure 4 depicts the results of immunohistochemical staining of the TFPI-2 protein. Fig. 4(A) depicts the results of imunohistochemical staining of human adult normal tissue sections with anti-TFPI-2 antibody. Strong staining was observed in placenta, a much weaker staining in liver and heart, and no staining was observed in brain, kidney, lung and skeletal muscle. Fig. 4(B) depicts the results of a cross-inhibition assay. Recombinant TFPI-2 protein inhibited TFPI-2 staining in placenta.
Figure 5 depicts the expression pattern of TFPI-2 protein in endometrial cysts examined by immunohistochemistry. Strong staining was observed for epithelial cells, and to some extent, stromal cells of endometrial cysts by anti-TFPI-2 antibody (left panels). No positive staining could be observed in the same endometrial cysts tissues with anti-rabbit IgG as the negative control (right panels). Upper panels show images with lower magnification (X100) and lower panels show those with higher magnification (X200). Arrows indicate epithelial cells of the cysts.

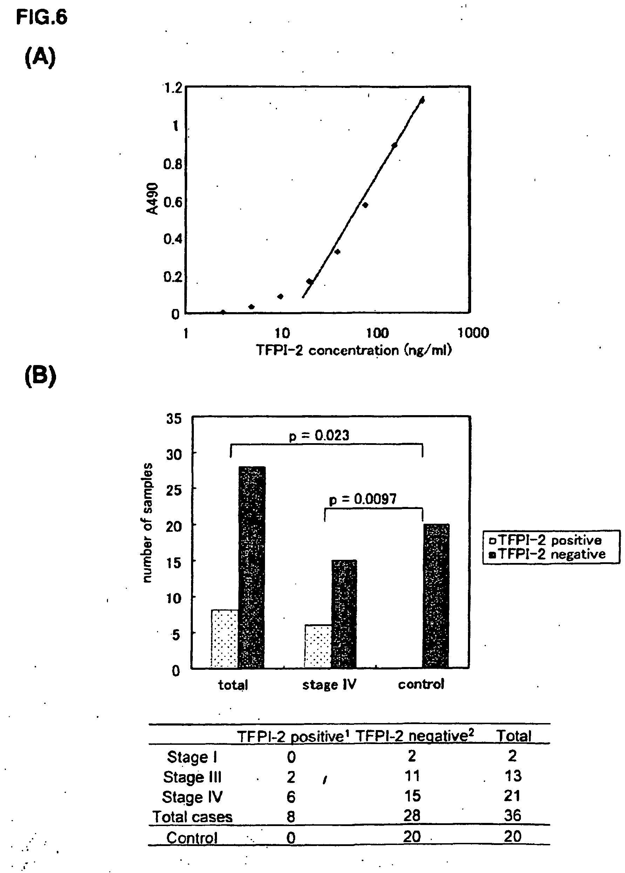

Figure 6(A) depicts the standard curve for TFPI-2 ELISA plotted using serially diluted recombinant TFPI-2. Each plotted point represents the mean value of duplicate measurements. Fig. 6(B) depicts a bar graph showing the result of detection of TRPI-2 in sera obtained from endometiosis patients and controls. TFPI-2 was detected in eight of 36 endometrial serum samples (p= 0.023), in six of 21 rASRM stage IV samples (p= 0.0097). No expression of TFPI-2 was detected in 20 control samples.

### DETAILED DESCRIPTION OF THE INVENTION

The words "a", "an" and "the" as used herein mean "at least one" unless otherwise specifically indicated.

To elucidate the nature of endometriosis, the present inventors carried out cDNA microarray analysis (Arimoto et al. (2003) Int. J. Oncol. 22:551-60). By comparing expression patterns between endometriotic tissues and corresponding eutopic endometria using genome-wide cDNA microarray analysis, the inventors identified several genes that were commonly up-regulated in endometrial cysts. One gene, encoding tissue factor pathway inhibitor-2 (TFPI-2) was discovered among this up-regulated gene. This gene shows relatively lower expression in normal human vital organ tissues and thus it is unlikely that this expression is essential for the maintenance or life. Therefore, suppressing the expression of this gene in an organism by targeting and such would not cause fatal side effects associated with the suppression of genes that are highly expressed in vital organs. In addition, TFPI-2 is known as secreted protein. Detection of secreted protein is relatively easier as compared to non-secreted proteins because antibodies against the proteins and biological samples such as peripheral blood, which is easier to collect than tissues, can be used for the detection.

Therefore, the inventors contemplated that this protein might function as preferable diagnostic markers for endometriosis. The expression level of TFPI-2 in placenta is the highest among normal human tissues. While TFPI-2 circulates in blood of normal men and non-pregnant women in extremely low concentrations, its level increases 40-fold to 70-fold in the plasma of pregnant women (Buztow et al. (1988) Clin. Chem. 34: 1591-3). The expression of TFPI-2 in endometrial cysts indicates that this protein may be secreted in the serum of endometriosis patients as well as in that of pregnant women. If this prediction is true, TFPI-2 may serve as a better marker for the diagnosis of endometriosis. Detection of this protein in the serum of endometriosis patients was expected to establish a new diagnostic method for the disease.

Thus, in this study, the present inventors established an enzyme-linked immunosorbent assay (ELISA) system to detect TFPI-2 protein in sera derived from patients with endometriosis, and demonstrated that the detected expression level ofTFPI-2 protein was elevated in sera from patients of advanced stages of endometriosis among the examined sera. This result suggested that the production and secretion into serum of TFPI-2 protein increases in response to disease progress. Hitherto, CAL25 was the most popular marker for endometriosis. However, as explained above, the specificity of CA125 as a marker for endometriosis is low and that of TFPI-2 protein seems to be higher, due to the detection of TFPI-2 only in sera from pregnant women and not in those from non-pregnant women and men. Accordingly, the findings of the present invention demonstrate that TFPI-2 protein is an effective marker for diagnosing endometriosis.

The present invention demonstrates the involvement of TFPI-2 protein in human ovarian endometriosis. Since the expression of this protein is relatively low in normal human adult tissues, gene encoding this protein has strong potential as target for therapy. In this study, the present inventors report that expression of this gene may play an important role in the progression or maintenance of endometriosis, and, thus, suggest that this gene product is promising target for the development of drugs for endometriosis.

### Diagnosing avarian endometriosis

The present application provides a method for diagnosing ovarian endometriosis. According to the present method, a subject suffering from ovarian endometriosis can be diagnosed. The subject to be diagnosed according to the present method is preferably a mammal including human, non-human primates (monkey, baboon, chimpanzee, etc.), mouse, rat, guinea-pig, rabbit, dog, cat, sheep, pig, horse and cow.

Ovarian endometriosis may be diagnosed by examining the expression of the TFPI-2 protein in a subject-derived biological sample. Specifically, the method of diagnosing ovarian endometriosis involves determining (measuring) the expression level of the TFPI-2 protein. The expression of the TFPI-2 protein may be determined and if desired, expression of this protein can be determined along with other proteins whose expression level is known to be altered according to conditions, *e.g*., ovarian endometriosis or non-ovarian endometriosis

According to the present method, the expression level of the TFPI-2 protein in a biological sample derived from a subject is compared to a control level of the same protein. The subject-derived biological sample may be any sample so long as the expression of the TFPI-2 protein can be detected in a patient of ovarian endometriosis and includes, but is not limited to, blood, serum and tissue samples obtained from a test subject. Preferable subject-derived biological samples include blood and serum, because such samples can be readily obtained from peripheral blood vessels.

The phrase "control level" refers to the expression level of the TFPI-2 protein in a reference sample. The reference sample should be the same type of sample as the biological sample examined in the present method of diagnosis. Namely, when the subject-derived biological sample is blood, then blood is used as a reference sample to determine the control level. The reference sample is derived from a subject or a population of subjects for whom the parameter to be compared is known, *i.e*., endometriotic or non-endometriotic. Alternatively, the control level may be calculated from a database of molecular information derived from subject(s) for which the assay parameter or condition is known.

According to the present method of diagnosing ovarian endometriosis, the expression level of the TFPI-2 protein in a subject-derived biological sample may be compared to multiple control levels of the protein. The control levels may be derived from biological samples with different known parameters (*i.e*., endometriotic or non-endometriotic). Thus, the expression level of the protein in a subject-derived biological sample may be compared to the control level corresponding to ovarian endometriosis patient (ovarian endometriosis control level), and then to the control level corresponding to non-ovarian endometriosis subject (normal control level). Thus, the control level may be a single expression pattern derived from a single reference population or may be a plurality of expression patterns. For example, the control level can be a database of expression patterns derived from previously tested samples.

Whether an expression level in a subject-derived biological sample as compared to that in a control level indicates ovarian endometriosis depends on the kind of the sample used for determining the control level. For example, when the control level is detected in a reference sample derived from a non-endometriotic individual, similar expression levels between the subject-derived biological sample and the reference sample indicates that the subject is non-endometriotic. Herein, such control levels are referred to as "normal control levels". A normal control levels indicates an expression level detected in a normal, healthy individual or in a population of individuals known not to be suffering from ovarian endometriosis. A normal healthy individual is one with no clinical symptoms of ovarian endometriosis. Conversely, when the control level is detected in a reference sample derived from an endometriotic patient, a similar expression profile between the subject-derived biological sample and the reference sample indicates that the subject suffers from ovarian endometriosis. Herein, such control levels are referred to as "ovarian endometriosis control levels" or "disease control levels". The phrase "ovarian endometriosis (or disease) control level" refers to the expression profile of the TFPI-2 protein found in a patient or a population of patients suffering from endometriosis.

The expression level of the TFPI-2 protein in a subject-derived biological sample is considered to be altered when the sample expression level differs from a control level by more than 0.1, 0.5, 1.0, 2.0, 5.0,10.0 or more folds. Alternatively, an expression level in a subject-derived biological sample increased by 1%, 5%, 10%, 25%, 50% or more as compared to a control level indicates alteration of the expression level in the subject sample.

An increase in the expression level of the TFPI-2 protein detected in a subject-derived biological sample as compared to a normal control level indicates that the subject suffers from ovarian endometriosis. In contrast, a similarity in the expression level of the TFPI-2 protein detected in a subject-derived biological sample as compared to an ovarian endometriosis control level indicates that the subject suffers from ovarian endometriosis.

Alteration in the expression of the TFPI-2 protein in a subject-derived biological sample as compared to a normal control level indicates that the subject suffers from ovarian endometriosis.

When required, comparison of the expression level in a subject-derived biological sample to a control level can be conducted with respect to a control protein whose expression is independent of the parameter or condition being measured. A "control protein" is one whose expression level is known not to differ between the endometriotic or non-endometriotic state of the individual to be tested. Expression levels of a control protein in the test and reference samples can be used to normalize the levels in the compared populations.

According to the method of the present application, protein expression levels can be determined by methods well known in the art, including, but not limited to, immunoassays using an antibody against the TFPI-2 protein. Suitable immunoassays for measuring the expression level of a protein include all kinds of known immunoassays, so long as it can be used for the detection of the TFPI-2 protein in a subject-derived biological sample, and include, but are not limited to, competitive assays and non-competitive assays; homogeneous assays and heterogeneous assays; homologous assays, heterologous assays and hetero-antibody assays; and assays wherein labeling substances, such as radioisotopes, enzymes, lanthanides, fluorescent substances, luminescent substances, free radicals and the like, are used. Competitive assays involve the competitive binding of labeled and non-labeled substances to a small amount of antibody. Exemplary competitive assays include, but are not limited to, radioimmunoassays (RIA) wherein the antigen is labeled with radioisotope; enzyme immunoassays (EIA) wherein enzymes are used as the labeling substance; time-resolved fluoroimmunoassays (TR-FIA), including, for example, dissociation-enhanced lanthanide flurorometric immunoassays (DELFIA^{®}) wherein lanthanides are used as the labeling substance; fluoroimmunoassays (FIA) using fluorescent substances that emit fluorescent upon irradiation with ultraviolet; and luminescence immunoassays (LIA) using chemical luminescent substances. In non-competitive assays, sandwich binding is performed wherein the target substance is exposed to excess antibody and then detected with labeled antibodies. Similarly to the competitive assays, the non-competitive assays can be also conducted using radioisotopes, enzymes, lanthanides, fluorescent substances, luminescent substances and such, and include, but are not limited to, immunoradiometric assays (IRMA), enzyme-linked ummunosorbent assays (ELISA), immunofluorometric assays (IFMA), time-resolved immunofluorometric assays (TR-IFMA), immunoluminometric assays (ILMA), and the like. In homogenous assays, measurement is conducted in a solution state, whereas in heterogeneous assays involve immobilizing the antibody on a solid matrix. The term "homologous assay" refers to an assay system wherein the target to be detected, standard sample and antibodies are all derived from the same species. Conversely, heterologous assays utilize an assay system for one animal species for the detection of antigens in an animal of another species (*e.g*., bovine against goat, rat against mouse, etc.). When the heterologous assay does not meet a good result, the use of homologous standard samples and heterologous antibodies are sometimes successful in RIA and such, which method is classified as a hetero-antibody assay.

According to the method of the present invention the expression level of the protein is determined by ELISA using a polyclonal antibody against the TFPI-2 protein as the labeled antibody, and a monoclonal antibody against the protein as the immobilized antibody. A small amount of the TFPI-2 protein in a subject-derived biological sample is expected to be efficiently detected by such a method.

The present application refers to the use of antibodies, particularly antibodies against the TFPI-2 protein or a fragment of such an antibody. As used herein, the term "antibody" refers to an immunoglobulin molecule having a specific structure that interacts (binds) specifically with a molecule comprising the antigen used for synthesizing the antibody (*i.e*., the TFPI-2 protein or fragments thereof) or with an antigen closely related to it. An antibody that binds to the TFPI-2 protein may be in any form, such as monoclonal or polyclonal antibodies, and includes antiserum obtained by immunizing an animal, such as a rabbit, with the polypeptide, all classes of polyclonal and monoclonal antibodies, human antibodies and humanized antibodies produced by genetic recombination.

Furthermore, the antibody may be a fragment of an antibody or a modified antibody, so long as it binds to its antigen (*i.e*., the TFPI-2 protein) and such binding can be detected. For instance, the antibody fragment may be Fab, F(ab')2, Fv or single chain Fv (scFv), in which Fv fragments from H and L chains are ligated by an appropriate linker (Huston et al. (1988) Proc. Natl. Acad. Sci. USA 85: 5879-83). More specifically, an antibody fragment may be generated by treating an antibody with an enzyme, such as papain or pepsin. Alternatively, a gene encoding the antibody fragment may be constructed, inserted into an expression vector, and expressed in an appropriate host cell (see, for example, Co et al. (1994) J. Immunol. 152: 2968-76; Better M. and Horwitz (1989) Methods Enzyme. 178:476-96; Pluckthun and Skerra (1989) Methods Enzyme. 178: 497-515; Lamoyi (1986) Methods Enzyme. 121: 652-63; Rousseaux et al. (1986) Methods Enzyme. 121:663-9; Bird and Walker (1991) Trends Biotechnol. 9:132-7).

An antibody may be modified by conjugation with a variety of molecules including but not limited to, matrices for immobilizing the protein and labels for detecting the antibody. Alternatively, the modified antibody can be obtained through chemical modification of the antibody. Such modification methods are conventional in the field.

An antibody may be directly or indirectly labeled with labeling substances, such as radioisotopes (³H, ¹⁴C, ³²P, ³³P, ³³S, ¹²⁵I, ¹³¹I, etc.), enzymes (alkaline phosphatase, peroxidase, β-glucuronidase, β-galactosidase, β-glucosidase, glucose oxidase, glucose-6-phosphate dehydrogenase, alcohol dehydrogenase, penicillinase, catalase, urease, luciferase, etc.), fluorescent substances (fluorescein isothiosyanete (FITC), rhodamine, etc.), lanthanides (europium (EU), etc.), luminescent substances (luminol, acridinium ester, etc.), free radicals (piperidine-N-oxide derivatives, pyrrolidine-N-oxide derivatives, oxazolidine-N-oxide derivatives, etc.) and biotin/avidin, by conventional methods. Antibodies may be directly labeled with the above labeling substances through the use of a cross-linker (*e.g*., N,N'-orthophenylene dimaleimide, 4,4'-dithiopyridine, etc.). Indirect labeling methods of antibodies include conjugating of the antibodies with small molecular weight haptens, such as biotin, dinitrophenyl and pyridoxal, wherein detection involves a substance that binds to the hapten(s).

When the antibody is labeled with a radioisotope, the detection or measurement can be carried out by liquid scintillation. Alternatively, antibodies labeled with enzymes can be detected or measured by adding a substrate of the enzyme to detect the enzymatic change of the substrate, such as generation of color, with absorptiometer. Alternatively, enzymes such as peroxidase may be used for the present method, and the peroxidase can be detected by reacting n-phenylenediamine under the existence of hydrogen peroxide and measuring the absorbance at a wave length of A₄₉₀ as described in Example 1 under the item of "Measurement of TFPI-2 concentration". Further, in cases where a fluorescent substance is used as the label, the bound protein may be detected or measured using a fluorophotometer. Furthermore, as described in Example 1 under the item of "Measurement of TFPI-2 concentration", a polyclonal antibody against the TFPI-2 protein, for ELISA may be biotinylated using, for example, the ECL Protein Biotinylation Module (Amersham Biosciences). Following binding with proteins in a biological sample, the biotin conjugated on the polyclonal antibody is reacted with avidin. Methods for measuring the avidins bound to biotins are known in the art and include methods wherein the avidin is conjugated with detectable enzymes. When lanthanides, such as Eu, are used as the labeling substance, the fluorescence (615 nm) emitted upon excitation with a light of a wavelength of 340nm can be detected. Kits using lanthanides are commercially available (*e.g*., those from PerkinElmer called Dissociation-enhanced lanthanide fluorometric immunoassay (DELFIA)). Free radical labels can be detected based on the change in the spectrum of its electron spin resonance (ESR).

Matrices to immobilize an antibody (polyclonal or monoclonal, though polyclonals are preferred for their highly efficient detection of antigens in a sample) are not restricted in any way and include, but are not limited to, matrices in the form of plates, beads, fibers, wells, etc. made of polystyrene, polyethyrene, polyvinyl chloride, polyester, nylon, polyacetal, fluoroplastic, glass, cellulose, agarose, mental, etc. The immobilization of an antibody to a matrix may be conducted according to any conventional chemical binding or physical adsorption methods. An antibody may be indirectly immobilized on a matrix using secondary antibodies recognizing antibodies (*e.g*., anti-IgG antibody), or protein A or protein G that binds to the constant region of an antibody.

### Assessing efficacy of treatment of ovarian endometriosis in a subject

The differentially expressed TFPI-2 protein identified herein also allows for the course of treatment of ovarian endometriosis to be monitored. According to the method of the present application, a biological sample, such as blood or serum, is obtained from a subject undergoing treatment for ovarian endometriosis. The method for assessment can be conducted in a manner analogous to the method of diagnosing ovarian endometriosis of the present invention described above.

Specifically, when required, biological samples can be obtained from the subject at various time points before, during or after the treatment. The expression level of the TFPI-2 protein in the biological sample is then determined and compared to a control level, for example, a reference sample derived from an individual whose state of ovarian endometriosis (*i.e*., endometriotic or non-endometriotic) is known. The control level is determined in a biological sample that has not been exposed to the treatment of interest.

When the control level is derived from a biological sample taken from an individual not suffering from ovarian endometriosis (i.e., a normal control level), a similarity between the expression level in the subject-derived biological sample and the normal control level indicates that the treatment is efficacious. A difference between the expression level of the TFPI-2 protein in the subject-derived biological sample and the normal control level indicates a less favorable clinical outcome or prognosis.

Furthermore, TFPI-2 protein expression levels determined in a subject-derived biological sample obtained after treatment (i.e., post-umiment levels) can be compared to TFPI-2 protein expression levels determined in a subject-derived biological sample obtained prior to treatment onset (i.e., pre-treatment levels). A decrease in expression levels of TFPI-2 in post-treatment samples also suggests that the treatment is efficacious.

As used herein, the term "efficacious" indicates that the treatment leads to a reduction in the expression of a pathologically up-regulated protein (the TFPI-2 protein) or a decrease in size, prevalence or proliferating potential of endometrial cysts in a subject. When a treatment is applied prophylactically, "efficacious" indicates that the treatment retards or prevents formation of ovarian endometrial cysts. The assessment of endometrial cysts can be made using standard clinical protocols.

The efficaciousness of a treatment is determined in association with any known method for diagnosing or treating ovarian endometriosis. Ovarian endometriosis is diagnosed for example, by identifying symptomatic anomalies, e.g., progressive dysmenorrhea, dyspareunia, chronic pelvic pain and infertility, along with surgical identification of endometrial glands and stroma outside the uterus.

### Assessing the prognosis of a subject with ovarian endometriosis

The present application further provides a method of assessing the prognosis of a subject with ovarian endometriosis by comparing the expression level of the TFPI-2 protein in a subject -derived biological sample, such as blood, to a control level. Alternatively, the expression level of the TFPI-2 protein in a biological sample derived from subjects may be measured over a spectrum of disease stages to assess the prognosis of the subject. The method for assessment can be conducted in a manner analogous to the method of diagnosing ovarian endometriosis of the present invention described above.

For example, an increase in the expression level of the TFPI-2 protein in the subject-derived sample as compared to a normal control level indicates a less favorable prognosis for the subject. Conversely, a similarity between the expression level of the TFPI-2 protein in the subject-derived sample as compared to a normal control level indicates a more favorable prognosis for the subject.

### Kit

The present application also provides a kit comprising any one or more of the materials (reagents and labware) used for performing the above-described method of diagnosing ovarian endometriosis. Preferably, the kit comprises a detection reagent, i.e., one or more antibodies binding to the TFPI-2 protein. The reagents such as antibodies (either bound to a solid matrix or packaged separately with reagents for binding them to the matrix), control reagents (positive and/or negative) and/or a means for detection of the antibody are preferably packaged in separate containers.

The assay format of the kit is not restricted in any way, so long as the TFPI-2 in a subject-derived biological sample can be detected with the use of the kit. Preferably, the kit is in the format of an immunoassay, encompassing any kind of immunoassay known in the art to detect a protein in a sample. For example, ELISA is a preferred method for performing the diagnosis. A kit for conducting ELISA preferably contains (1) a detectably labeled polyclonal antibody binding to the TFPI-2 protein; and (2) a monoclonal antibody binding to the TFPI-2 protein immobilized on a solid matrix. Alternatively, the kit may preferably contains (1) a detectably labeled polyclonal antibody binding to the TFPI-2 protein; (2) a monoclonal antibody binding to the TFPI-2 protein that can be immobilized on a solid matrix; and (3) a reagent for immobilizing the antibody of (2) on a matrix. The kit may further comprise a matrix for immobilizing the antibody of (2) with the reagent of (3).

A preservative such as Thimerosal may be added to the enzyme-labeled antibodies included in the kit. Further, stabilizers, such as glycerol, may be added. Labeled antibodies can be stored long term by subjecting to lyophilization and keeping them under cool and dark conditions. A preservative, such as sodium azide, may be added to the immobilized antibody and the antibody is preferably stored under a cool condition.

Furthermore, control samples (with a normal control level and/or ovarian endometriosis control level) may also be included in the kit. The normal control sample can be obtained from a healthy subject and the endometriosis control sample also can be obtained from a subject which was diagnosed endometriosis. Alternatively, endometriosis control sample can be prepared adding TFPI-2 protein to normal control sample. Moreover, reagents (substrate, etc.) for detecting the labeled antibody are included in the kit Preferably, instructions (written, tape, VCR, CD-ROM, etc.) for carrying out the assay may also be included in the kit.

Various gynecological studies about TFPI-2 have been performed in terms of its association with the progression of pregnancy. However, to date, there has been no report that this protein relates to the development of endometriosis. Accondingly, the present inventors examined whether TFPI-2 is secreted from cells through the establishment of stable transformants of the gene, and demonstrated that this gene was overexpressed in endometrial cysts by semi-quantitative RT-PCR and immunohistochemistry (Fig. 1 and 5).

As discussed in detail below in the Examples section, the present invention demonstrates the possible involvement of the TFPI-2 protein in human ovarian endometriosis. Since the expression of this transcripts is relatively low in normal human adult tissues, this gene itsself can serve as novel targets for therapy.

The gene-expression analysis of ovarian endometriosis described herein, obtained through a combination of laser-capture dissection and genome-wide cDNA microarray, has identified specific genes as targets for prevention and therapy of ovarian endometriosis. Based on the expression of the TFPI-2 protein, the present application provides molecular diagnostic markers for identifying or detecting ovarian endometriosis.

The methods described herein are also useful in the identification of additional molecular targets for prevention, diagnosis and treatment of ovarian endometriosis. The data reported herein add to a comprehensive understanding of ovarian endometriosis, facilitate development of novel diagnostic strategies and provide clues for identification of molecular targets for therapeutic drugs and preventative agents. Such information contributes to a more profound understanding of ovarian cadometriosis, and provides indicators for developing novel strategies for diagnosis, treatment and ultimately prevention of ovarian endometriosis.

The following examples are presented to illustrate the present invention and to assist one of ordinary skill in making and using the same. The examples are not intended in any way to otherwise limit the scope of the invention.

### [Example 1] Materials and general methods

### 1. Tissue preparation

Endometrial cysts were obtained after informed pre-operative consent from 6 patients who underwent cystectomy at the Department of Obstetrics and Gynecology. For immunohistochemistry, tissue sections were mounted in O.C.T compound (Sakura Finetechnical) immediately after resection.

### 2. Sera

Sera were obtained from 36 patients one day before laparoscopic operation. Two of the patients were classified as in the rASRM stage I, 13 in stage **III** and 21 in stage IV. As controls, scrum samples were collected from 12 apparently healthy women and eight men between the ages of 24 to 40.

### 3. Semi-quantitative RT-PCR

Semi-quantitative RT-PCR experiments were conducted as described previously (Ono et al. (2000) Cancer Res. 60: 5007-11). Endometrial cysts were obtained to extract total RNA, and T7-based RNA amplificalion with the total RNA was performed according to a previous study (Arimoto et al. (2003) Int. J. Oncol. 22: 551-60). A3-µg aliquot of amplified-RNA from each sample was reverse-transcribed for single-stranded cDNAs using random primer (Roche) and Superscript II (Life Technologies, Inc.). Each cDNA mixture was diluted for subsequent PCR amplification with the following primer sets
- TFPI-2 forward primer: 5'-TGACAGCATGAGGAAACAAATC-3'(SEQ ID NO: 1) and reverse primer. 5'-ACGACCCCAAGAAATGAGTG-3' (SEQ ID NO: 2);
- -G3PDH forward primer: 5'-CGACCACTTTGTCAAGCTCA-3' (SEQ ID NO: 3) and reverse primer: 5'-GGTTGAGCACAGGGTACTTTATT-3' (SEQ ID NO: 4)).
The expression of G3PDH served as an internal control. PCR reactions were optimized for the number of cycles to ensure product intensity within the linear phase of amplification.

### 4. Northern blotting

Multiple-tissue Northern blot membranes containing 2-µg of poly(A)⁺ RNA from various human tissues (Clontech) were hybridized with ³²P-labeled partial cDNA fragments of TFPI-2 (TFPI-2 forward primer. 5'-GGAAAATTCGGAAGAAGCAA-3' (SEQ ID NO: 5) and reverse primer. 5'-ACGACCCCAA GAAATGAGTG-3' (SEQ ID NO: 2);. Conditions of hybridization and washing are described elsewhere (Nakagawa et aL (2000) Oncogene 19: 210-6).

### 5 Recombinant protein production in Escherichia coli and polyclonal antibody generation

Recombinant proteins were prepared from human cDNA clones encoding TFPI-2 (GenBank Accession No. D29992) without their signal peptides (TFPI-2: N-terminal 22 residues) by inserting into the *E*. *call* expression vector pET28a (Novagen) and transforming into BL21-CodonPlus^{®} (DE3)-RIL competent cells (Stratagene). Protein expression was induced by 0.5mM isopropyl β-D-thiogalactoside (IPTG) with incubation at 37°C for 3 h, and cells were harvested by centrifugation. *E*. *coli* cell pellets that expressed recombinant TFPI-2 was dissolved in 100mM sodium phosphate dehydrate (pH 8.0) containing 6M guanidine hydrochloride, 10mM Tris and 10mM imidazole. Histidine-tagged TFPI-2 protein was purified by BD TALON™ Metal Affinity Resins (BD Biosciences). Then, 6 M guanidine hydrochloride was replaced with 8M urea. These purified recombinant proteins were refolded by dilution and reducing the concentration of urea to 4M. This protein solution was injected into rabbits every week, and after 10 times of immunization, antiscrum was collected (MBL), The specific antibodies were isolated by affinity chromatography using Affi-Gel 10 (Bio-Rad) to which purified recombinant protein is bound covalently.

### 6. Cell lines

Human endometrial adenocarcinoma cell line, HEC-151, was provided by Kitasato University (Sagamihara, Japan) and was maintained in Eagle's minimal essential medium with 10% fetal bovine serum (FBS). Human glioma cell line, Hs.683, was purchased from the American Type Culture Collection (Manassas, VA, USA). Hs.683 and COS-7 cells were maintained in Dulbeoco's modified Eagle's medium with 10% FBS. Cells were maintained at 37°C in an atmosphere of humidified air with 5% CO₂.

### 7. Western blotting

Samples were resolved by SDS-PAGE under reducing conditions and transferred onto Hybond™ ECL™ Nitrocellulose membranes (Amersham Pharmacia Biotech). The blotted membranes were blocked with Block Ace™ powder (Dainippon Seiyaku) and treated with rabbit anti-TFPI-2 (034 µg/ml) specific polyclonal antibodies. Afler washing, the blots were treated with horseradish peroxidase-conjugated donkey anti-rabbit IgG (Amersham Biosciences) and developed with enhanced chemilumine scence (ECL; Amersham Biosciences).

### 8. Immunofluorescent staining

Cells were replated on Lab-Tek® II Chamber Slide System (Nalge Nunc International) followed by fixation with 4% paraformaldehyde in PBS and permeabilization with 0.1 % Triton X-100 in PBS for 3 min at 4°C. After blocking with 3% BSA in PBS for I h at room temperature, the cells were incubated with rabbit anti-TFPI-2 (0.34 µg/ml) antibodies for 1 h at room temperature. These antibodies were stained with goat anti-rabbit secondary antibody conjugated to rhodamine, respectively, and viewed with a BX51 microscope (Olympus). As described in the following stable transformants section, stable transformants were also incubated with mouse anti-myc 9E10 monoclonal antibody (Santa Cruz Biotechnology, 0.2µg/ml) and stained with rabbit anti-mouse secondary antibody conjugated to FTPC.

### 9. Immunohistochemical staining and cross-inhibition assay

Archieved, paraformaldehyde-fixed, paraffin-embedded tissue sections were purchased from Biochain bistitute, Inc. Sections were stained with DAKO En Vision™+

System, HRP (DAB) (Dako) according to manufacturer's protocol. The polyclonal antibodies were used at 2.5 µg/ml (anti-TFPI-2 antibody). For immunostaining inhibition, antibodies were pre-incubated overnight at 4°C with the corresponding recombinant proteins that were used as the antigen (1.5 µg).

### 10. Construction of stable transformants

COS-7 cells were seeded and transfected with pcDNA3. 1/myc-His(-)-TFPI-2 (Invitrogen), or with an empty vector as a control, using FuGENE6 Transfection Reagent (Roche) according to the manufacturer's protocol. Cells were cultured for up to 3 weeks in the medium containing 0.4mg/ml of G418. Individual clones were isolated with cloning cylinders. The cell clones that expressed TFPI-2 (as confirmed by RUT-PER Western blotting, and immunofluorescent staining) were maintained in the medium containing 0.4mglml of G418 and used for further investigations.

### 11. Measurement of TFPI-2 concentration.

The concentration of TFPI-2 was measured by ELISA using biotinylated anti-TFPI-2 polyclonal antibody. First, 100µl rabbit anti-TFPI-2 antibody (10µg/ml in 50 mmol/l sodium carbonate, pH 9.5) was added to each well of 96-well microtitration plates (Maxisorp Immunoplate, Nunc) and incubated overnight at 4°C. After washing the plates three times with PBS-0.1% Tween 20, each of the wells was blocked with 200µl PBS-2% HSA at 37°C for 2 h. Then, the plates were washed again three times with PBS-Tween 20. Subsequently, a 100µl serum sample was added to each of the wells and the plates were incubated at 37°C for 2 h. The plates were then washed three times with PBS-Tween 20 and 100µl biotin-labeled anti-TFPI-2 antibody (1µg/ml in PBS-1% BSA) was added to each of the wells. Biotinylation of the antibody was performed using ECL Protein Biotinylation Module (Amersham Biosciences) according to the manufacturer's protocol. After incubation for 2h at 37°C, the plates were washed three times with PBS-Tween 20 and then treated with 100µl peroxidase-conjugated avidin (DAKO, diluted 4000-fold with PBS-1% BSA) for 2 h at room temperature. Then, the plates were washed six times with PBS-Tween 20 and 100µl *o*-phenylenediamine (DAKO, 4 tablets in 12ml distilled water and 5µl of 30% hydrogen peroxide) was added to each of the wells. After incubating the plates for an adequate time (3 to 5 min) at room temperature, 100µl of 0.5 mol/l surfuric acid was added to each of the wells and the absorbance (A₄₉₀) was measured. The concentration of TFPI-2 in samples was interpolated from standard curves of A₄₉₀ versus recombinant TFPI-2 concentration.

### [Example 2] Confirmation of expression of TFPI-2 in endometriosis by semi- quantitative RT-PCR

cDNA microarray was used to analyze gene-expression profiles of 23,040 genes in ovarian endometrial cysts from 23 patients (Arimoto et al. (2003) Int. J. Oncol. 22: 551-60). Among the up-regulated genes, the gene encoding the TFPI-2 protein, which was found to be overexpressed in all of 9 informative cases whose signal intensities of the gene were higher than the cut-off in the secretory phase and down-regulated in more than 50% of the cases in the proliferative phase, was focused and selected for further study. Furthermore, semi-quantitative RT-PCR analyses were performed to confirm the elevated expression of TFPI-2 in the secretory phase (Fig. 1).

### [Example 3] The expression of the TFPI-2 protein

To investigate whether TFPI-2 is secreted protein as described previously, a population of mammalian cells that stably overexpress this protein was established by transfecting pcDNA3.1(-)-*TFPI-*2-myc-his into COS7 cells. Expression and subcellular localization in some transformant was confirmed by Western blotting (Fig. 2A) and immunofluorescent staining (Fig. 2B). As described previously, prominent TFPI-2 triplet bands (approximately 33, 31 and 27 kDa) were observed for the TFPI-2 sense transformants with anti-TFPI-2 polyclonal antibody, and corresponding TFPI-2 proteins were also detected in culture medium (Sense), but not in the vector transfectants (Mock) (Fig. 2A; left panel). In addition, endogenous expression of the TFPI-2 protein in HEC-151 and Hs.683 cells detected by Western blotting (Fig. 2A, right panel) and immunofluorescent staining (Fig. 2C) with anti-TFPI-2 polyclonal antibody revealed that the TFPI-2 protein localizes in the cytoplasm like a secreted protein. These results suggest that TFPI-2 is secreted protein.

### [Example 4] Detection of TFPI-2 protein expression in endometrial cysts and normal human tissues by immunohistochemistry

TFPI-2 was initially demonstrated to be specifically expressed in the placenta (Fig. 3, upper panel) by Northern blot analysis. Next, immunohistochemical staining was performed on human normal tissues, and the TFPI-2 protein was demonstrated to be expressed in the syncytiotrophoblasts and decidual cells of placenta; weak expression was found in the cytoplasm of hepatocytes around the vein and cardiac muscle cells, and almost no staining (i.e., expression) was observed in brain, kidney, lung and skeletal muscle (Fig. 4A).

Furthermore, to investigate whether TFPI-2 antibody can specifically recognize the corresponding proteins in placenta or small intestine, a cross-inhibition assay was performed. As a result, reduced staining of these cells showed that both polyclonal antibodies reacted specifically to their corresponding protein (Fig. 4B).

Moreover, expression of the TFPI-2 protein in the sections of endometrial cysts was investigated by immunohistochemistry. Positive staining for the protein was observed in epithelial cells of endometrial cysts by anti-TFPI-2 antibody (Fig. 5, left and middle panels), whereas no positive staining in the same enodometrial cysts tissues could be observed using anti-rabbit IgG as the negative control.

### [Example 5] Detection of the TFPI-2 protein in endometriotic sera

To further examine the possibility whether TFPI-2 serves as a diagnostic marker for endometriosis, an ELISA assay using specific TFPI-2 polyclonal antibodies was conducted to measure the concentration of the TFPI-2 protein in sera. The dose-response curve obtained according to the assay was linear over a range of 20 to 320 ng/ml of TFPI-2 (Fig. 6A). Using this standard curve, the concentration of the TFPI-2 protein in sera of patients with endometriosis detected by ELISA was calculated. It has been reported that the concentration of TFPI-2 in sera from both men and non-pregnant women was less than 1 ng/ml (Buztow et al. (1988) Clin. Chem. 34: 1591-3). Further, based on this reported concentration, the dose-response curve was confirmed to be linear within the range encompassing the concentration measured for eight healthy men and twelve healthy women studied as controls in this study. In eight of 36 endometrial serum samples, the level of TFPI-2 was > 20ng/ml (range 262-66.3ng/ml, median 31.0ng/ml), whereas that in all of 20 control samples was < 20ng/ml (Fig. 6B, p= 0.023). In addition, the TFPI-2 protein was detected in six of 21 sera from rASRM stage IV patients (p= 0.0097).

### Industrial Applicability

The present invention involves the discovery that TFPI-2 level is elevated in the sera of endometriosis patients as compared to normal controls. Accordingly, the TFPI-2 gene and protein find utility as novel diagnostic markers (i.e. blood or serum). Using the level of TFPI-2 as an index, the present invention provides a method for diagnosing a endometriosis patient.

While the invention has been described in detail and with reference to specific embodiments thereof it is to be understood that the foregoing description is exemplary and explanatory in nature and is intended to illustrate the invention and its preferred embodiments. Through routine experimentation, one skilled in the art will readily recognize that various changes and modifications can be made therein without departing from the scope of the invention. Thus, the invention is intended to be defined not by the above description, but by the following claims.

## Claims

1. An in vitro method of diagnosing ovarian endometriosis in a subject, comprising the steps of:
(1) determining the expression level of a tissue factor pathway inhibitor-2 (TFPI-2) protein in a blood or blood derived sample obtained from a subject, and
(2) comparing the expression level determined in step (1) with a control level, wherein an increase in expression level as compared to a normal control level indicates that said subject suffers from ovarian endometriosis and/or a similarity of said expression level as compared to a disease control level indicates that said subject suffers from ovarian endometriosis
wherein the expression level is determined by ELISA, wherein a polyclonal antibody and a monoclonal antibody against the TFPI-2 protein is used as the labeled antibody and immobilized antibody, respectively.

2. Use of a kit for the in vitro diagnosis of ovarian endometriosis , wherein the kit comprises
(1) a detectably labelled antibody binding to the TFPI-2 protein, and
(2) an antibody binding to the TFPI-2 protein that is immobilized on a solid matrix.

3. The use of claim 2, wherein the detectably labeled antibody is polyclonal and the antibody immobilized on a solid matrix is monoclonal.

4. The use of a kit for the in vitro diagnosis of ovarian endometriosis, wherein the kit comprises
(1) a detectably labeled antibody binding to the TFPI-2 protein,
(2) an antibody binding to the TFPI-2 protein which may be immobilized on a matrix, and
(3) a reagent for immobilizing the antibody of (2) on a matrix.

5. The use of claim 4, wherein the detectably labeled antibody is polyclonal and the antibody that may be immobilized on a solid matrix is monoclonal.

6. The use of claim 2, wherein the kit further comprises either or both of normal control sample and endometriosis control sample.

7. The use of claim 6, wherein the control sample is blood or blood derived sample.

## Patentansprüche

1. *In vitro*-Verfahren zur Diagnose von Ovarialendometriose in einem Individuum, umfassend die Schritte:
(1) das Bestimmen des Expressionsspiegels eines Tissue Factor Pathway Inhibitor-2 (TFPI-2)-Proteins in einer Blut- oder von Blut stammenden Probe, die von einem Individuum erhalten wurde, und
(2) das Vergleichen des Expressionsspiegels, der in Schritt (1) bestimmt wurde, mit einem Kontrollspiegel, wobei eine Erhöhung des Expressionsspiegels verglichen mit einem normalen Kontrollspiegel anzeigt, dass das Individuum an Ovarialendometriose leidet, und/oder eine Ähnlichkeit des Expressionsspiegels verglichen mit einem Spiegel einer Erkrankungskontrolle anzeigt, dass das Individuum an Ovarialendometriose leidet,
wobei der Expressionsspiegel mittels ELISA bestimmt wird, wobei ein polyclonaler Antikörper und ein monoclonaler Antikörper gegen das TFPI-2-Protein als der markierte bzw. der immobilisierte Antikörper verwendet wird.

2. Verwendung eines Kits zur *in vitro*-Diagnose von Ovarialendometriose, wobei der Kit umfasst:
(1) einen nachweisbar markierten Antikörper, der an das TFPI-2-Protein bindet, und
(2) einen Antikörper, der an das TFPI-2-Protein bindet, der an einer festen Matrix immobilisiert ist.

3. Verwendung nach Anspruch 2, wobei der nachweisbar markierte Antikörper polyclonal ist und der Antikörper, der an einer festen Matrix immobilisiert ist, monoclonal ist.

4. Verwendung eines Kits zur *in vitro*-Diagnose von Ovarialendometriose, wobei der Kit umfasst:
(1) einen nachweisbar markierten Antikörper, der an das TFPI-2-Protein bindet,
(2) einen Antikörper, der an das TFPI-2-Protein bindet, der an einer festen Matrix immobilisiert sein kann, und
(3) ein Reagens zur Immobilisierung des Antikörpers von (2) an eine Matrix.

5. Verwendung nach Anspruch 4, wobei der nachweisbar markierte Antikörper polyclonal ist und der Antikörper, der an einer festen Matrix immobilisiert ist, monoclonal ist.

6. Verwendung nach Anspruch 2, wobei der Kit zudem eine normale Kontrollprobe oder eine Endometriose-Kontrollprobe oder beides umfasst.

7. Verwendung nach Anspruch 6, wobei die Kontrollprobe eine Blut- oder von Blut stammende Probe ist.

## Revendications

1. Méthode *in vitro* de diagnostic d'endométriose ovarienne chez un sujet, comprenant les étapes de :
(1) détermination du niveau d'expression d'une protéine inhibiteur de la voie du facteur tissulaire-2 (TFPI-2) dans un échantillon de sang ou dérivé du sang obtenu à partir d'un sujet, et
(2) comparaison du niveau d'expression déterminé à l'étape (1) à un niveau de contrôle,
dans laquelle une augmentation du niveau d'expression par rapport à un niveau de contrôle normal indique que ledit sujet souffre d'endométriose ovarienne et/ou une similarité dudit niveau d'expression par rapport à un niveau de contrôle de la maladie indique que ledit sujet souffre d'endométriose ovarienne,
dans laquelle le niveau d'expression est déterminé par test ELISA, dans lequel un anticorps polyclonal et un anticorps monoclonal contre la protéine TFPI-2 sont utilisés respectivement en tant qu'anticorps marqué et anticorps immobilisé.

2. Utilisation d'un kit pour le diagnostic in vitro de l'endométriose ovarienne, dans laquelle le kit comprend
(1) un anticorps marqué de façon détectable qui se lie à la protéine TFPI-2, et
(2) un anticorps qui se lie à la protéine TFPI-2 qui est immobilisé sur une matrice solide.

3. Utilisation selon la revendication 2, dans laquelle l'anticorps marqué de façon détectable est polyclonal et l'anticorps immobilisé sur une matrice solide est monoclonal.

4. Utilisation d'un kit pour le diagnostic *in vitro* de l'endométriose ovarienne, dans laquelle le kit comprend
(1) un anticorps marqué de façon détectable qui se lie à la protéine TFPI-2,
(2) un anticorps qui se lie à la protéine TFPI-2 qui peut être immobilisé sur une matrice, et
(3) un réactif pour immobiliser l'anticorps de (2) sur une matrice.

5. Utilisation selon la revendication 4, dans laquelle l'anticorps marqué de façon détectable est polyclonal et l'anticorps qui peut être immobilisé sur une matrice solide est monoclonal.

6. Utilisation selon la revendication 2, dans laquelle le kit comprend en outre l'un ou les deux parmi un échantillon de contrôle normal et un échantillon de contrôle d'endométriose.

7. Utilisation selon la revendication 6, dans laquelle l'échantillon de contrôle est un échantillon de sang ou dérivé du sang.
